(19) Europäisches Patentamt
European Patent Office
Office européen des brevets

(11) Veröffentlichungsnummer: **0 023 653**
**B1**

(12) # EUROPÄISCHE PATENTSCHRIFT

(45) Veröffentlichungstag der Patentschrift: **29.09.82**

(51) Int. Cl.³: **A 61 K 31/41** // C07D249/08, C07C49/175

(21) Anmeldenummer: **80104240.9**

(22) Anmeldetag: **18.07.80**

(54) **1,2,4-Triazolylenolether enthaltende pharmazeutische Zusammensetzungen und deren Herstellung.**

(30) Priorität: **04.08.79 DE 2931756**

(43) Veröffentlichungstag der Anmeldung: **11.02.81 Patentblatt 81/6**

(45) Bekanntmachung des Hinweises auf die Patenterteilung: **29.09.82 Patentblatt 82/39**

(84) Benannte Vertragsstaaten: **AT BE CH DE FR GB IT LI NL SE**

(56) Entgegenhaltungen:
**DE-A-2 600 799**
**DE-A-2 720 868**
**DE-A-2 720 949**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Draber, Wilfried, Dr., In den Birken 81, D-5600 Wuppertal-1 (DE)**
Erfinder: **Büchel, Karl Heinz, Prof. Dr., Bergerheide 62, D-5600 Wuppertal-1 (DE)**
Erfinder: **Plempel, Manfred, Dr., Pahlkestrasse 5, D-5090 Wuppertal-1 (DE)**
Erfinder: **Haller, Ingo, Dr., Viktoriastrasse 99, D-5600 Wuppertal-1 (DE)**

**EP 0 023 653 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 1,2,4-Triazolylenolether enthaltende pharmazeutische Zusammensetzungen und deren Herstellung

Die Erfindung betrifft 1,2,4-Triazolylenolether enthaltende pharmazeutische Zusammensetzungen und deren Herstellung.

Es ist bereits bekannt geworden, dass 1,2,4-Triazolylether, wie beispielsweise substituierte 1-Phenoxy-2-(R-oxy)-3,3-dimethyl-1-(1,2,4-triazol-1-yl)-butane, gut antimykotische Eigenschaften aufweisen (vergleiche DE-OS 27 20 868). Die Wirkung dieser Verbindungen ist jedoch nicht immer ganz befriedigend.

Es wurde gefunden, dass die neuen 1,2,4-Triazolyl-enolether der allgemeinen Formel I

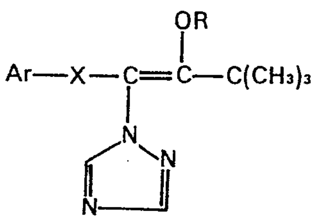 (I)

in welcher

Ar für gegebenenfalls einfach oder zweifach durch Halogen, Alkyl mit 1-3 Kohlenstoffatomen, Trifluormethyl, Nitro, Phenyl oder Chlorphenyl substituiertes Phenyl,
R für Alkyl mit 1-4 Kohlenstoffatomen, und
X für Sauerstoff oder Methylen steht,

und deren physiologisch verträglichen Säureadditions-Salze und Metallsalz-Komplexe gute antimykotische Eigenschaften aufweisen.

Die erfindungsgemäss zu verwendenden Verbindungen der allgemeinen Formel kommen in den geometrischen Isomeren E (trans) und Z (cis) vor. Bei der E,Z-Nomenklatur werden die an der Doppelbindung stehenden Substituenten nach der Cahn-Ingold-Prelog-Regel nach abnehmender Priorität eingeordnet. Stehen die bevorzugten Substituenten auf derselben Seite der Doppelbindung, ist die Konfiguration Z (abgeleitet von zusammen), stehen sie auf entgegengesetzter Seite, E (abgeleitet von entgegen). Sowohl die einzelnen Isomeren als auch die Gemische werden erfindungsgemäss beansprucht.

Überraschenderweise zeigen die erfindungsgemäss verwendbaren 1,2,4-Triazolylenolether der allgemeinen Formel I eine bessere antimykotische Wirksamkeit als die aus dem Stand der Technik bekannten 1,2,4-Triazolylether, die chemisch und wirkungsgemäss naheliegende Verbindungen sind. Die erfindungsgemäss verwendbaren Stoffe stellen somit eine Bereicherung der Pharmazie dar.

Besonders bevorzugt sind diejenigen 1,2,4-Triazolylenolether der allgemeinen Formel, in denen Ar für Phenyl steht, das gegebenenfalls einfach oder zweifach, gleich oder verschieden substituiert ist durch Fluor, Chlor, Brom, Jod, Methyl, Ethyl, Isopropyl, Trifluormethyl, Nitro, Phenyl oder Chlorphenyl; R für Methyl, Ethyl, Isopropyl, Isobutyl oder tert.-Butyl steht; und X die in der Erfindungsdefinition angegebene Bedeutung hat.

Im einzelnen seien ausser den bei den Herstellungsbeispielen genannten Verbindungen die folgenden Verbindungen der allgemeinen Formel I genannt:

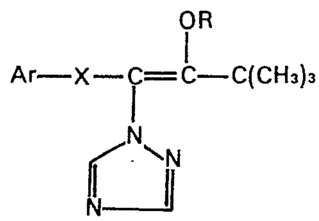 (I)

| Ar | X | R |
|---|---|---|
| 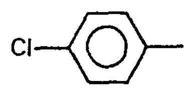 | O | i-$C_4H_9$ |
| 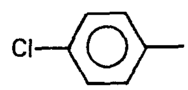 | $CH_2$ | $CH_3$ |
| 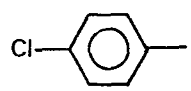 | $CH_2$ | i-$C_3H_7$ |
| 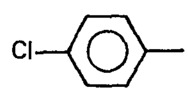 | $CH_2$ | i-$C_4H_9$ |
| 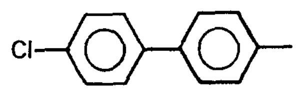 | O | $CH_3$ |
| 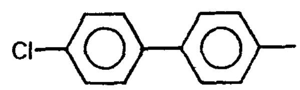 | O | $C_2H_5$ |
| 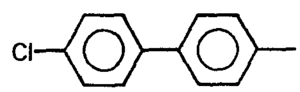 | $CH_2$ | $CH_3$ |
| 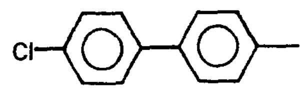 | $CH_2$ | $C_2H_5$ |
| 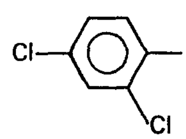 | O | $CH_3$ |
| 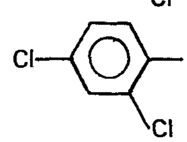 | O | $C_2H_5$ |
| 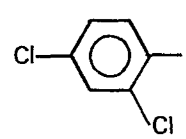 | $CH_2$ | $CH_3$ |

| Ar | X | R |
|---|---|---|
| 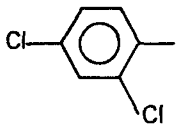 | $CH_2$ | $C_2H_5$ |
| 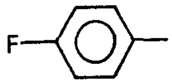 | O | $CH_3$ |
| 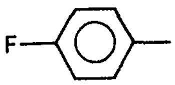 | O | $C_2H_5$ |
| 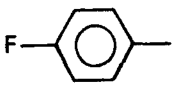 | $CH_2$ | $CH_3$ |
| 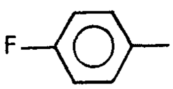 | $CH_2$ | $C_2H_5$ |
| 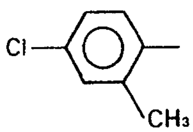 | O | $CH_3$ |
| 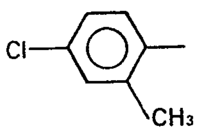 | O | $C_2H_5$ |
| 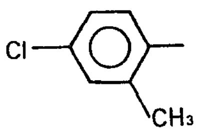 | $CH_2$ | $CH_3$ |
| 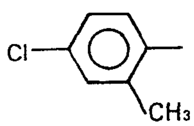 | $CH_2$ | $C_2H_5$ |
| 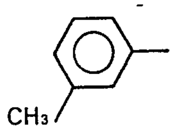 | O | $CH_3$ |
| 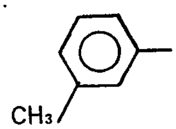 | O | $C_2H_5$ |
| 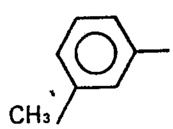 | $CH_2$ | $CH_3$ |
| 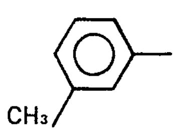 | $CH_2$ | $C_2H_5$ |
| 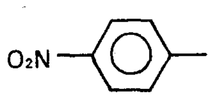 | O | $CH_3$ |
| 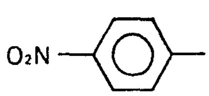 | O | $C_2H_5$ |
| 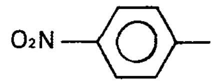 | $CH_2$ | $CH_3$ |
| 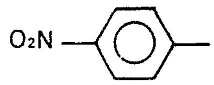 | $CH_2$ | $C_2H_5$ |
| 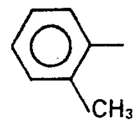 | O | $CH_3$ |
| 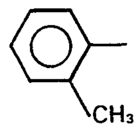 | O | $C_2H_5$ |
| 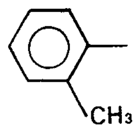 | $CH_3$ | $CH_3$ |
| 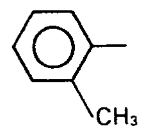 | $CH_3$ | $C_2H_5$ |
| 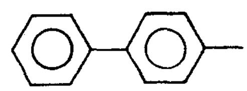 | O | $CH_3$ |
| 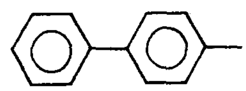 | O | $C_2H_5$ |
| 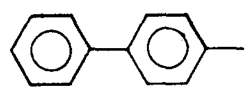 | $CH_2$ | $CH_3$ |
| 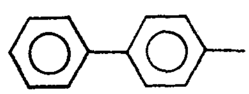 | $CH_2$ | $C_2H_5$ |

Die erfindungsgemäss zu verwendenden Wirkstoffe, deren Säureadditions-Salze und Metallsalz-Komplexe sind noch nicht bekannt. Sie können jedoch gemäss einem eigenen Vorschlag hergestellt werden, indem man 1,2,4-Triazolyl-ketone der allgemeinen Formel II

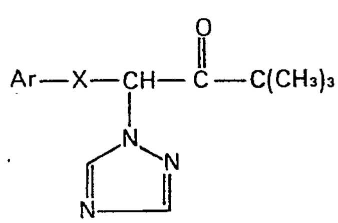 (II)

in welcher
Ar und X die oben angegebene Bedeutung haben,

mit Alkylsulfaten, wie z.B. Dimethyl- oder Diethylsulfat, bzw. Alkylhalogeniden, wie insbesondere Alkylbromiden und -jodiden, in Gegenwart

eines inerten organischen Lösungsmittels, wie beispielsweise Dimethylsulfoxid, bei Temperaturen zwischen 0 und 100°C umsetzt.

In einer bevorzugten Ausführungsform wird die erfindungsgemässe Umsetzung in einem Zweiphasensystem, wie beispielsweise wässrige Natron- oder Kalilauge/Toluol oder Methylenchlorid, gegebenenfalls unter Zusatz von 0,1–1 Mol eines Phasen-Transfer-Katalysators, wie beispielsweise Ammonium- oder Phosphoniumverbindungen, beispielsweise seien Benzyl-dodecyl-dimethyl-ammoniumchlorid (Zephirol) und Triethyl-benzyl-ammoniumchlorid genannt, durchgeführt (vergleiche auch die Herstellungsbeispiele).

Die Verbindungen der allgemeinen Formel I fallen in Form der geometrischen Isomerengemische an. Die Isolierung der einzelnen geometrischen Isomeren erfolgt nach üblichen Methoden, wie z.B. aufgrund unterschiedlicher Löslichkeit, durch Salzbildung, durch Craig-Verteilung oder durch chromatographische Trennverfahren bzw. durch Kombination dieser Methoden. Eine eindeutige Strukturzuordnung erfolgt aufgrund der $^1$H-NMR-Daten, insbesondere unter Verwendung von Verschiebungsreagenzien. Die durch diese Reagenzien bewirkten Signalverschiebungen sind um so grösser, je kleiner der räumliche Abstand zwischen dem komplexierten Zentralatom des Verschiebungsreagenzes und dem betrachteten Proton ist (vergleiche auch die Herstellungsbeispiele).

Die 1,2,4-Triazolyl-ketone der allgemeinen Formel II sind bekannt (vergleiche DE-AS 2201063 und DE-OS 2734426) und können nach den dort angegebenen Verfahren erhalten werden, indem man z.B. entsprechende Halogenketone mit 1,2,4-Triazol in Gegenwart eines Verdünnungsmittels und in Gegenwart eines Säurebinders umsetzt (vergleiche auch die Herstellungsbeispiele).

Die ausserdem für das erfindungsgemässe Verfahren als Ausgangsstoffe benötigten Alkylsulfate bzw. -halogenide sind allgemein bekannte Verbindungen der organischen Chemie.

Zur Herstellung von physiologisch verträglichen Säureadditionssalzen der Verbindungen der allgemeinen Formel I kommen vorzugsweise folgende Säuren in Frage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure. Die Säureadditions-Salze der Verbindungen der allgemeinen Formel I können in einfacher Weise nach üblichen Salzbildungsmethoden z.B. durch Lösen einer Verbindung der allgemeinen Formel I in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel I kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. Chlorwasserstoffsäure und Bromwasserstoffsäure, ferner Phosphorsäure, Salpetersäure und Schwefelsäure.

Die Metallsalz-Komplexe von Verbindungen der allgemeinen Formel I können in einfacher Weise nach üblichen Verfahren erhalten werden, so z.B. durch Lösen des Metallsalzes in Alkohol, z.B. Ethanol und Hinzufügen zur Verbindung der allgemeinen Formel I. Man kann Metallsalz-Komplexe in bekannter Weise, z.B. durch Abfiltrieren, Isolieren und gegebenenfalls durch Umkristallisation reinigen.

Die erfindungsgemäss verwendbaren Verbindungen der allgemeinen Formel I, ihre Säureadditions-Salze und Metallsalz-Komplexe weisen antimikrobielle, insbesondere starke antimykotische Wirkungen auf. Sie besitzen ein sehr breites antimykotisches Wirkungsspektrum, insbesondere gegen Dermatophyten und Sprosspilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sprosspilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur Erfindung gehören pharmazeutische Zubereitungen, die neben nicht toxischen, inerten pharmazeutisch geeigneten Trägerstoffen einen oder mehrere erfindungsgemässe Wirkstoffe enthalten oder die aus einem oder mehreren erfindungsgemässen Wirkstoffen bestehen sowie Verfahren zur Herstellung dieser Zubereitungen.

Zur Erfindung gehören auch pharmazeutische Zubereitungen in Dosierungseinheiten. Dies bedeutet, dass die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärke, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quartenäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gelees können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isotearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süssmittel, z.B. Sacharin enthalten.

Die therapeutisch wirksamen Verbindungen sollen in den oben aufgeführten pharmazeutischen Zubereitungen vorzugsweise von etwa 0,5 bis 95 Gew.-% der Gesamtmischung vorhanden sein.

Die oben aufgeführten pharmazeutischen Zubereitungen können ausser den erfindungsgemässen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemässen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Beispiel A
Antimykotische in-vitro-Wirksamkeit

Versuchsbeschreibung:
Die in-vitro-Prüfung wurden im Reihenverdünnungstest mit Keiminokula von durchschnittlich 5 × 10 Keimen/ml Substrat durchgeführt. Als Nährmedium dienten

a) für Dermatophyten und Schimmelpilze:
Sabourand's milieu d'épresive
b) für Hefen
Isotonic Sensitest-Broth von Oxid.

Die Bebrütungstemperatur betrug 28°C; Bebrütungszeit war 24 Stunden bei Hefen und 96 Stunden bei Dermatophyten und Schimmelpilzen.

In diesem Versuch zeigen die erfindungsgemässen Verbindungen sehr gute MHK-Werte.

Beispiel B
Antimikrobielle in-vivo-Wirksamkeit (oral) bei Mäuse-Candidose

Versuchsbeschreibung:
Mäuse vom Typ SPF-$CF_1$ wurden intravenös mit 1-2 × $10^6$ logarithmisch wachsenden Candida-Zellen, die in physiologischer Kochsalzlösung suspendiert waren, infiziert. Eine Stunde vor und sieben Stunden nach der Infektion werden die Tiere mit jeweils 50-100 mg/kg Körpergewicht der Präparate oral behandelt.

Ergebnis:
Unbehandelte Tiere starben 3 bis 6 Tage post infectionem. Die Überlebensrate am 6. Tag post infectionem betrug bei unbehandelten Kontrolltieren etwa 5%.

In diesem Versuch erweisen sich die erfindungsgemässen Verbindungen als sehr gut wirksam, wobei sie bekannte Verbindungen übertreffen.

## Herstellungsbeispiele

### Beispiel 1

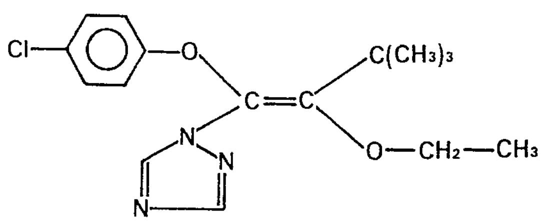

(E)-Form

Zu 29,5 g (0,1 Mol) 1-(4-Chlorphenoxy)-3,3-dimethyl-1-(1,2,4-triazol-1-yl)-butan-2-on, 16 g (0,1 Mol) Ethyljodid und 0,5 g Benzyl-dodecyl-dimethyl-ammoniumchlorid (Zephirol) in 50 ml Dimethylsulfoxid werden bei Raumtemperatur langsam 4,4 g (0,11 Mol) Natronlauge, in wenig Wasser gelöst, zugetropft. Die ablaufende Reaktion ist leicht exotherm (bis ca. 50°C). Danach lässt man 3 Stunden bei Raumtemperatur nachrühren, gibt das Reaktionsgemisch auf Wasser, neutralisiert mit Essigsäure und extrahiert mit Methylenchlorid. Die organische Phase wird mit Wasser ausgeschüttelt, über Natriumsulfat getrocknet und eingeengt. Nach Trennung durch Craig-Verteilung erhält man 6,0 g (22% der Theorie) 1-(4-Chlorphenoxy)-3,3-dimethyl-2-ethoxy-1-(1,2,4-triazol-1-yl)-1-buten als Isomerengemisch (Z/E = 1/3). Durch präparative HPLC erhält man die (Z)- und die (E)-Form des 1-(4-Chlorphenoxy)-3,3-dimethyl-2-ethoxy-1-(1,2,4-triazol-1-yl)-1-butens, wobei die (E)-Form einen Schmelzpunkt von 57-62°C besitzt.

Die Strukturzuordnung der beiden Isomeren erfolgt mittels $^1$H-NMR-Spektroskopie unter Verwendung von $Eu(TFC)_3$ = Europium-tris-[3-trifluormethylhydroxymethylen)-d-camphorato] als Verschiebungsreagenz. Die Messungen werden in $CDCl_3$ durchgeführt.

Tabelle: Shiftwerte und Verschiebungsdifferenzen der Resonanzsignale mit und ohne Verschiebungsreagenz (VR)

| Fragment: | (Z)-Isomeres | | | (E)-Isomeres | | |
|---|---|---|---|---|---|---|
| | δ (ppm) ohne VR: | δ (ppm) mit VR: | Δ S | δ (ppm) ohne VR: | δ (ppm) mit VR: | Δ S |
| $C(CH_3)_3$ | 1,00 | 2,77 | 1,77 | 1,15 | 2,16 | 1,01 |
| $CH_3$ | 1,22 | 1,80 | 0,58 | 1,02 | 2,70 | 1,68 |
| $CH_2$ | 4,02 | 5,10 | 1,08 | 3,33 | 6,45 | 3,12 |
| Triaz.-H | 7,94 | >12 | >4 | 7,91 | >12 | >4 |
| | 8,10 | | | 8,31 | | |
| arom. H | 7,02 | 7,80 | 0,78 | 6,92 | 7,58 | 0,66 |
| | 7,25 | 8,60 | 1,35 | 7,19 | 8,46 | 1,27 |

Herstellung des Ausgangsproduktes

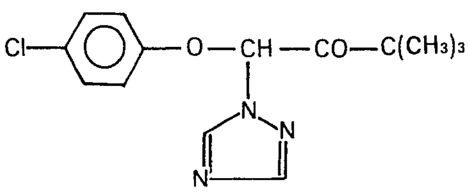

418 g (6,6 Mol) 1,2,4-Triazol werden in 3000 ml Aceton gelöst. Hierzu werden 934 g (7,2 Mol) wasserfreies, pulverisiertes Kaliumcarbonat gegeben, die Suspension zum Sieden erhitzt und eine Lösung von 1565 g (6 Mol) 1-(4-Chlorphenoxy)-1-chlor-3,3-dimethyl-butan-2-on in 1500 ml Aceton so zugetropft, dass die Mischung ohne Beheizung unter Rückfluss siedet. Nach beendeter Zugabe wird zur Vervollständigung der Reaktion 15 Stunden unter Rückfluss erhitzt; anschliessend wird der erhaltene Niederschlag abfiltriert, mit Aceton gewaschen und verworfen. Das Filtrat wird im Wasserstrahlvakuum vom Lösungsmittel befreit, der Rückstand in 3000 ml Toluol aufgenommen und einmal mit einer Lösung von 100 g 37%iger Salzsäure in 2000 ml Wasser gewaschen. Die wässerige Phase wird abgetrennt und verworfen; die organische Phase wird mit 5000 ml Wasser gewaschen und – nach Zugabe von weiteren 4000 ml Toluol – mit einer Lösung von 145 g Natriumhydroxid in 3500 ml Wasser 6 Stunden bei Raumtemperatur gerührt. Danach wird die organische Phase abgetrennt, mit Wasser neutral gewaschen und im Wasserstrahlvakuum vom Lösungsmittel befreit. Man erhält 1535 g (87% der Theorie) 1-(4-Chlorphenoxy)-3,3-dimethyl-1-(1,2,4-triazol-1-yl)-butan-2-on vom Schmelzpunkt 75–76°C.

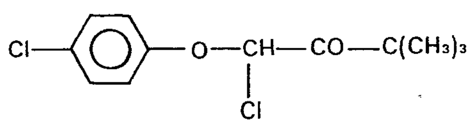

771 g (6 Mol) 4-Chlorphenol werden in 3600 ml Aceton gelöst. Man trägt 3 g wasserfreies Natriumjodid sowie 910 g (6,6 Mol) wasserfreies, pulverisiertes Kaliumcarbonat ein und lässt unter Rückfluss 895 g (6,3 Mol) 94,6%iges Monochlorpinakolin zutropfen. Nach 20 stündigem Rühren bei Rückflusstemperatur wird der Niederschlag abfiltriert, mit Aceton gewaschen und verworfen. Das Filtrat wird im Wasserstrahlvakuum vom Lösungsmittel befreit. Der resultierende weisse Rückstand wird mit 3000 ml Tetrachlorkohlenstoff aufgenommen und auf 60°C erwärmt. Zu dieser Lösung werden 891 g (6,6 Mol) Sulfurylchlorid ohne weitere Erwärmung so zugetropft, dass eine stetige Gasentwicklung erfolgt. Nach beendeter Zugabe wird 15 Stunden unter Rückfluss erhitzt. Schliesslich wird das Lösungsmittel im Wasserstrahlvakuum abdestilliert. Man erhält in quantitativer Ausbeute 1565 g 1-(4-Chlorphenoxy)-1-chlor-3,3-dimethyl-butan-2-on, das ohne weitere Reinigung zur oben beschriebenen Umsetzung verwendet werden kann.

Beispiel 2

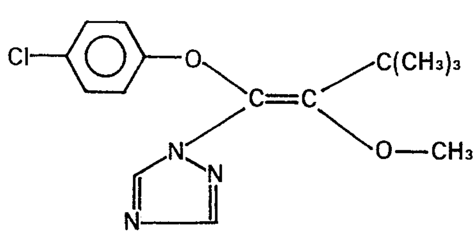

(E)-Form

Zu 146,5 g (0,5 Mol) I-(4-Chlorphenoxy)-3,3-dimethyl-1-(1,2,4-triazol-1-yl)-butan-2-on in 250 ml Dimethylsulfoxid werden 30 g Kaliumhydroxid, in wenig Wasser gelöst, zugetropft. Man lässt 1 Stunde nachrühren und tropft anschliessend 65 g (0,51 Mol) Dimethylsulfat zu. Dabei wird die Temperatur der Reaktionsmischung mittels Eisbad auf ca. 40°C gehalten. Man lässt 2 Stunden nachrühren, versetzt mit Wasser, saugt den kristallinen Niederschlag ab und kristallisiert aus Petrolether um. Man erhält 51 g (33% der Theorie) (E)-1-(4-Chlorphenoxy)-3,3-dimethyl-2-methoxy-1-(1,2,4-triazol-1-yl)-1-buten vom Schmelzpunkt 134–138°C.

In entsprechender Weise werden die nachfolgenden Beispiele der allgemeinen Formel I

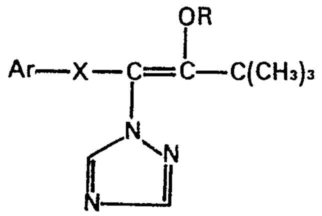

(I)

erhalten:

| Bsp. Nr. | Ar | X | R | Schmelzpunkt (°C) |
|---|---|---|---|---|
| 3 | 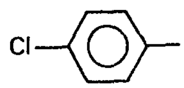 | O | i-$C_3H_7$ | 129–32 (E-Form) |
| 4 | 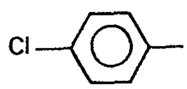 | $CH_2$ | $C_2H_5$ | 103-05 (Z-Form) |
| 5 | 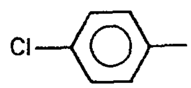 | $CH_2$ | $C_2H_5$ | $n_D^{22}$=1,5370 (E-Form) |

**Patentansprüche**

1. Antimykotisches Mittel, gekennzeichnet durch einen Gehalt an mindestens einem 1,2,4-Triazolylenolether der allgemeinen Formel I

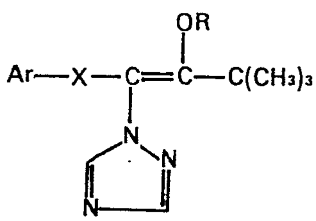

(I)

in welcher
Ar für gegebenenfalls einfach oder zweifach durch Halogen, Alkyl mit 1–3 Kohlenstoffatomen, Trifluormethyl, Nitro, Phenyl oder Chlorphenyl substituiertes Phenyl,
R für Alkyl mit 1–4 Kohlenstoffatomen und
X für Sauerstoff oder Methylen steht,
und/oder deren physiologisch verträglichen Säureadditions-Salze und/oder Metallsalz-Komplexe.

2. Antimykotisches Mittel nach Anspruch 1, gekennzeichnet durch einen Gehalt an 1-(4-Chlorphenoxy)-3,3-dimethyl-2-ethoxy-1-(1,2,4-triazol-1-yl)-1-buten.

3. Antimykotisches Mittel nach Anspruch 1, gekennzeichnet durch einen Gehalt an 1-(4-Chlorphenoxy)-3,3-dimethyl-2-methoxy-1-(1,2,4-triazol-1-yl)-1-buten.

4. Antimykotisches Mittel nach Anspruch 1, gekennzeichnet durch einen Gehalt an 1-(4-Chlorphenoxy)-3,3-dimethyl-2-isopropoxy-1-(1,2,4-triazol-1-yl)-1-buten.

5. Antimykotisches Mittel nach Anspruch 1, gekennzeichnet durch einen Gehalt an 1-(4-Chlorphenylmethyl)-3,3-dimethyl-2-ethoxy-1-(1,2,4-triazol-1-yl)-1-buten.

6. Verfahren zur Herstellung eines antimykotischen Mittels, dadurch gekennzeichnet, dass man 1,2,4-Triazolylenolether gemäss der allgemeinen Formel I in Anspruch 1 mit inerten, nichttoxischen, pharmazeutisch geeigneten Trägerstoffen vermischt.

**Claims**

1. Antimycotic agent, characterised in that it contains at least one 1,2,4-triazolyl-enol ether of the general formula I

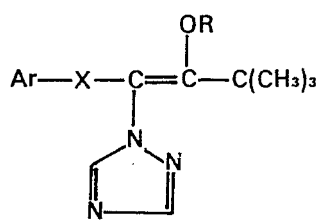

(I)

in which
Ar represents phenyl which is optionally monosubstituted or disubstituted by halogen, alkyl with 1–3 carbon atoms, trifluoromethyl, nitro, phenyl or chlorophenyl,
R represents alkyl with 1–4 carbon atoms and
X represents oxygen or methylene,

and/or physiologically acceptable acid addition salts and/or metal salt complexes thereof.

2. Antimycotic agent according to Claim 1, characterised in that it contains 1-(4-chlorophenoxy)-3,3-dimethyl-2-ethoxy-1-(1,2,4-triazol-1-yl)-1-butene.

3. Antimycotic agent according to Claim 1, characterised in that it contains 1-(4-chlorophenoxy)-3,3-dimethyl-2-methoxy-1-(1,2,4-triazol-1-yl)-1-butene.

4. Antimycotic agent according to Claim 1, characterised in that it contains 1-(4-chlorophenoxy)-3,3-dimethyl-2-isopropoxy-1-(1,2,4-triazol-1-yl)-1-butene.

5. Antimycotic agent according to Claim 1, characterised in that it contains 1-(4-chlorophenylmethyl)-3,3-dimethyl-2-ethoxy-1-(1,2,4-triazol-1-yl)-1-butene.

6. Process for the preparation of an antimycotic agent, characterised in that 1,2,4-triazolyl-enol ethers according to the general formula I in Claim 1 are mixed with inert, non-toxic, pharmaceutically suitable excipients.

**Revendications**

1. Agent antimycotique, caractérisé en ce qu'il contient au moins un 1,2,4-triazolylénoléther de formule générale I:

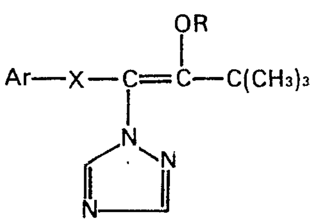

(I)

dans laquelle

Ar représente un groupe phényle éventuellement substitué une ou deux fois par un atome d'halogène, par un groupe alkyle contenant 1 à 3 atomes de carbone, par un groupe trifluorométhyle, par un groupe nitro, par un groupe phényle ou par un groupe chlorophényle,

R représente un groupe alkyle contenant 1 à 4 atomes de carbone, et

X représente un atome d'oxygène ou un groupe méthylène,

et/ou ses complexes de sels métalliques et/ou ses sels d'addition d'acide physiologiquement compatibles.

2. Agent antimycotique suivant la revendication 1, caractérisé en ce qu'il contient du 1-(4-chlorophénoxy)-3,3-diméthyl-2-éthoxy-1-(1,2,4-triazol-1-yl)-1-butène.

3. Agent antimycotique suivant la revendication 1, caractérisé en ce qu'il contient du 1-(4-chlorophénoxy)-3,3-diméthyl-2-méthoxy-1-(1,2,4-triazol-1-yl)-1-butène.

4. Agent antimycotique suivant la revendication 1, caractérisé en ce qu'il contient du 1-(4-chlorophénoxy)-3,3-diméthyl-2-isopropoxy-1-(1,2,4-triazol-1-yl)-1-butène.

5. Agent antimycotique suivant la revendication 1, caractérisé en ce qu'il contient du 1-(4-chlorophénylméthyl)-3,3-diméthyl-2-éthoxy-1-(1,2,4-triazol-1-yl)-1-butène.

6. Procédé de préparation d'un agent antimycotique, caractérisé en ce qu'on mélange des 1,2,4-triazolylénoléthers répondant à la formule générale I suivant la revendication 1 avec des substances supports inertes, non toxiques et pharmaceutiquement appropriées.